**Europäisches Patentamt**

**European Patent Office**

**Office européen des brevets**

(11) Veröffentlichungsnummer: **0 152 510**
**B1**

(12) **EUROPÄISCHE PATENTSCHRIFT**

(45) Veröffentlichungstag der Patentschrift:
25.11.87

(21) Anmeldenummer: **84101920.1**

(22) Anmeldetag: **23.02.84**

(51) Int. Cl.⁴: **E 04 B 1/70,** A 61 N 1/16

(54) **Gerät zur Entfeuchtung von Mauerwerk.**

(43) Veröffentlichungstag der Anmeldung:
28.08.85 Patentblatt 85/35

(45) Bekanntmachung des Hinweises auf die Patenterteilung:
25.11.87 Patentblatt 87/48

(84) Benannte Vertragsstaaten:
AT BE CH DE FR GB IT LI LU NL SE

(56) Entgegenhaltungen:
GB-A-1 407 151
US-A-4 418 481

DEUTSCHE BAUZEITSCHRIFT, Nr. 2, Februar 1980,
Seiten 249-257, 927-950, Gütersloh, DE; H.W. TENGE:
"Elektrophysikalische Verfahren zur
Mauertrockenlegung, Teil I,II"

(73) Patentinhaber: **Terramundo Ltd, P.O. Box 31
Templar House Don Road, St. Helier Jersey
Channel Islands (GB)**

(72) Erfinder: **Wehrli, Walter, Sigmundstrasse 14, CH-
9444 Diepoldsau (CH)**

(74) Vertreter: **Blum, Rudolf Emil Ernst, c/o E. Blum &
Co Patentanwälte Vorderberg 11, CH- 8044 Zürich
(CH)**

**Beschreibung**

Die Erfindung bezieht sich auf ein Gerät zur Entfeuchtung von Mauerwerk bei im Mauerwerk aufsteigender Feuchtigkeit, welches Gerät Kondensatoren und zu Spulen gewundene elektrische Leiter aufweist, wobei die beiden Pole eines jeweiligen Kondensators mit je einem Ende eines jeweiligen zur Spule gewundenen elektrischen Leiters verbunden sind.

Im Mauerwerk von Gebäuden werden durch aufsteigende Feuchtigkeit, hervorgerufen z. B. durch Grundwasser, Stauwasser, Sickerwasser oder unterirdisch fliessende Gewässer beträchtliche Schäden verursacht.

Da alle Baustoffe mehr oder weniger porös sind, saugen sie auf Grund ihrer Kapillarität das Wasser an, so dass dieses innerhalb der Mauern hochsteigen kann.

Der offene Kreislauf von Wasser in einer nassen Mauer, bedingt durch den osmotischen Druck, wird von unten nach oben und hier durch Verdunsten nach aussen geführt. Durch diesen Wassertransport wird ein elektrisches Feld in Richtung der steigenden Feuchtigkeit aufgebaut, dieses Feld wirkt als Pumpe.

Feuchtes Mauerwerk führt nicht nur zu bautechnischen Schäden wie teilweisen Zerfall, kristallinen Ausblühungen, Krustenbildung etc., sondern kann auch zu Erkrankungen von Menschen führen, die sich in Bauten mit feuchtem Mauerwerk aufhalten, z. B. Rheuma, Asthma, Infektionskrankheiten, etc., und vor allem entsteht auch ein sehr unwohnliches Raumklima in solchen Bauten.

Es ist bekannt, in Mauern aufsteigende Feuchtigkeit durch einlegen von Isolierlagen zu bekämpfen. Weiter sind verschiedene sogenannte Elektroosmoseverfahren bekannt, welche meistens beträchtliche bauliche Massnahmen bedingen, nicht an jeder Stelle und ohne Schwierigkeiten durchführbar sind (Bohren in Hauswänden), und in vielen Fällen in keinem Verhältnis zum erzielten Erfolg stehen.

Bewegen sich die Oberflächen zweier Stoffe aneinander vorbei (Reibung), laden sich die beiden Stoffe an ihren Grenzschichten entgegengesetzt elektrisch auf. Hat dabei der eine Stoff eine grössere elektrische Leitfähigkeit als der andere Stoff, so lädt sich der erste positiv, der zweite negativ elektrisch auf.

Bewegt sich Wasser in kapillarem Mauerwerk, so ist seine elektrische Ladung jener des Mauerwerks entgegengesetzt. Es entsteht zwischen den beiden Grenzschichten eine elektrische Potentialdifferenz, das ZETA-POTENTIAL. Das Wasser wird dabei durch die entgegengesetzten elektrischen Ladungen in den Kapillaren hochgezogen und benetzt somit weite Bereiche des Mauerwerkes. Es sind jedoch noch weitere Faktoren mitbestimmend, unter anderem die im Wasser gelösten Salze, die Luftionisation, etc. In der Fachwelt wird angenommen, dass alle diese Faktoren von den Wasserverhältnissen im Erdboden beeinflusst werden, weil unterirdisch strömendes Wasser bekanntlich elektrische und magnetische Felder zur Folge hat, die in ihrer Stärke stark schwanken, wodurch die elektrische Leitfähigkeit des Wassers verändert wird. Als weitere Ursachen wirken vor allem diese natürlichen Reizfelder und vermehrt auch durch technische Anlagen (z. B. erdverlegte Hochspannungsleitungen etc.) entstehende, sogenannte zivilisatorische Reizfelder.

Diese Reizfelder verlaufen je nach geophysikalischen Verhältnissen in weit ausgedehnten Bereichen, bzw. Reizzonen. Verläuft nun eine solche Zone durch ein Gebäude, wird das Reizfeld stark inhomogen. In elektrisch isolierendem Mauerwerk ist die Feldstärke grösser als in dem von ihm umschlossenen Innenraum, welche Tatsache auf Grund der stark dielektrischen Eigenschaften von Wasser zur Transport der Wassermoleküle führt, entweder im Mauerwerk selbst kapillar aufsteigend oder durch Diffusion aus der Luft zum Mauerwerk. Dieses ist die Ursache der Feuchtigkeitsbildung.

Was die physikalische Natur der Reizfelder betrifft, hegt die Fachwelt nach neuesten Erkenntnissen die Vermutung, dass eine Reizzone eine Region darstellt, in der starke Aktivität von stillen atmosphärischen Entladungen herrscht. Diese Entladungen haben den Charakter von Ladungsfluktuationen beziehungsweise dipolartigen Anregungszuständen, deren Beschreibung in den Bereich der Quantenphysik fällt. Sie scheinen dem Mechanismus der Pause bei atmosphärischen Blitzentladungen verwandt zu sein.

Es ist ein Entfeuchtungsgerät gemäss dem Oberbegriff des Patentanspruches 1 bekannt (US-A-4 418 481), bei dem zwei Spulen vorhanden sind, die mit jeweils einem Kondensator verbunden sind, wobei die Längsachsen der Kondensatoren die Achsen der mit ihnen verbundenen Spulen kreuzen. Dabei laufen die Achsen der Spulen senkrecht zueinander. Mit diesem Gerät lässt sich nur das elektrische Erdfeld beeinflussen. Es hat sich in der Praxis gezeigt, dass das Gerät das Aufsteigen von Feuchtigkeit in Mauerwerk verhindern kann. Eine genaue, abschliessende technische Erklärung für diesen Effekt kann noch nicht gemacht werden, es wird jedoch vermutet, dass dieses Gerät auf Eigenheiten, bzw. Veränderungen des elektrischen bzw. magnetischen Erdfeldes anspricht und seinerseits einen beeinflussenden Faktor bildet, welcher der im Mauerwerk aufsteigenden Bodenfeuchtigkeit entgegenwirkt. Dieses bekannte Gerät benötigt keine Energiezufuhr ausser jener des Erdfeldes.

Die Praxis hat nun aber gezeigt, dass es immer wieder Orte gibt, an denen das bekannte Gerät nur teilweise funktioniert. Insbesondere an Orten, an denen nur relativ schwache Erdfeldanomalien oder vorwiegend Anomalien des magnetischen Erdfeldes vorhanden waren, brachte dieses Gerät meist nicht den gewünschten Erfolg.

Hier will die Erfindung Abhilfe schaffen. Die Erfindung, wie sie in den Ansprüchen gekennzeichnet ist, löst die Aufgabe, ein Gerät zur Entfeuchtung von Mauerwerk zu schaffen, welches in seiner Wirksamkeit erheblich gesteigert ist, und vor allem auch an den Orten mit Erfolg eingesetzt werden kann, an denen die bekannten Geräte nicht oder mindestens nicht zur vollen Zufriedenheit funktionierten.

Die durch die Erfindung erreichten Vorteile sind om wesentlichen darin zu sehen, dass das Gerät auch an den Orten mit relativ schwachen örtlichen Erdfeldanomalien und vor allem völlig unabhängig von der Konstellation der vorhandenen Störfelder, d.h. diese können magnetischer, elektromagnetischer oder elektrischer Natur oder als Wechselwirkung dieser Kräfte bebildet sein, mit vollem Erfolg eingesetzt werden kann.

Im folgenden wird die Erfindung anhand mehrerer Ausführungswege darstellender Zeichnungen erläutert. Es zeigt:

Fig. 1 einen Schnitt entlang der Linie I-I der Fig. 2, wobei ein erster Ausführungsweg gezeichnet ist;

Fig. 2 einen Schnitt entlang der Linie II-II der Fig. 1 der Darstellung eines ersten Ausführungsweges;

Fig. 3 eine Darstellung gleich der Fig. 2 eines zweiten Ausführungsweges mit drei Spulen; und

Fig. 4 eine Darstellung gleich der Fig. 1 eines dritten Ausführungsweges mit gedruckten Schaltungen für die Spulen.

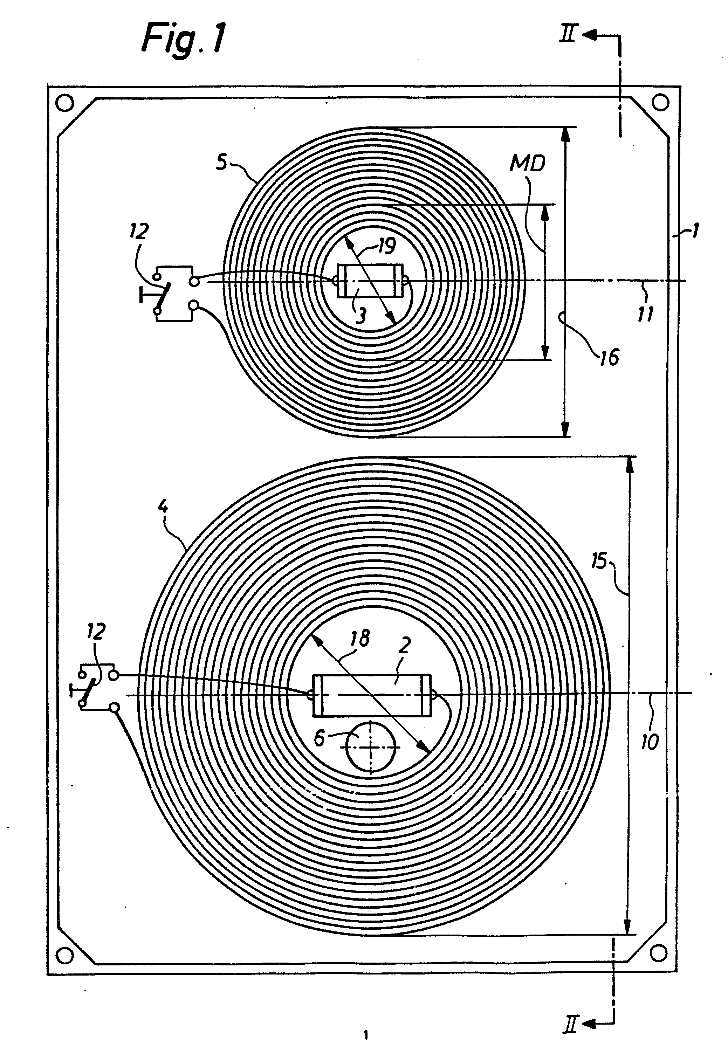

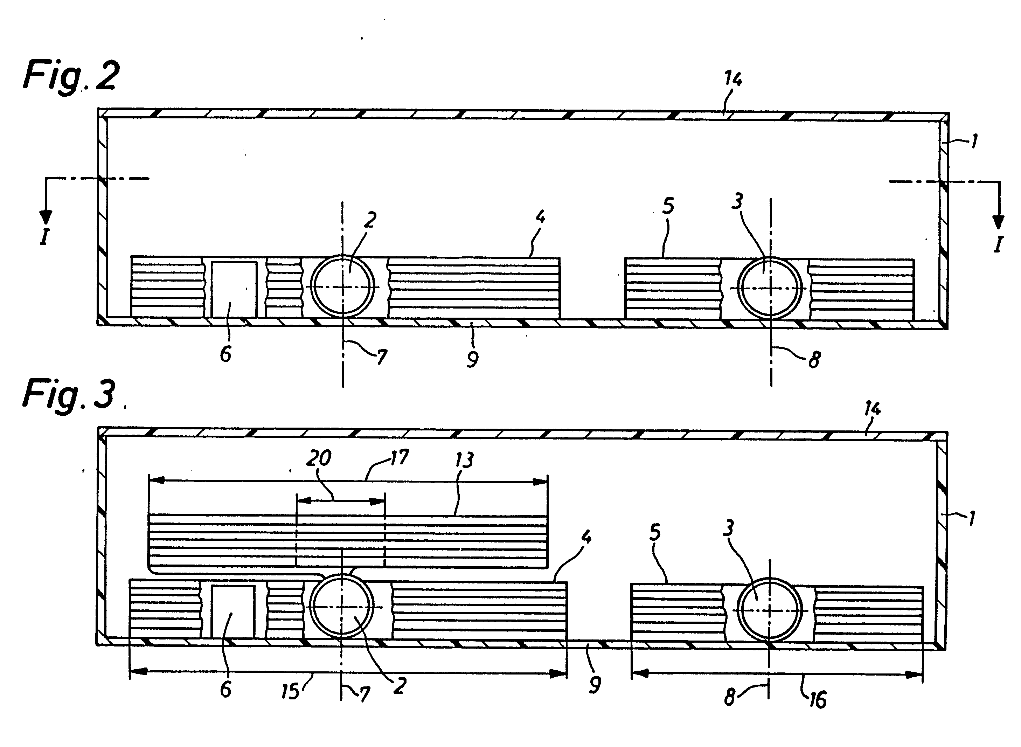

Das Gerät weist ein Gehäuse aus einem nicht elektrisch leitenden Stoff auf, welches Gehäuse in allen Figuren mit der Bezugsziffer 1 bezeichnet ist. Im Gehäuse 1 sind nach dem in der Figuren 1 und 2 gezeichneten Ausführungsweg ein erster Kondensator 2 und ein zweiter Kondensator 3 angeordnet. Weiter sind ein erster, zu einer ersten Spule 4 gewundener Leiter und ein zweiter, zu einer zweiten Spule 5 gewundener Leiter vorhanden. Der erste Kondensator 2 ist einerends mit dem einen Ende und andererends mit dem anderen Ende der ersten Spule 4 verbunden, und der zweite Kondensator 5 ist einerends mit dem einen Ende und andererends mit dem anderen Ende der zweiten Spule 5 verbunden. Damit ergeben sich zwei Schwingkreise begründende Schaltungen. In der Mitte der ersten Spule 4 ist neben dem ersten Kondensator 2 ein Permanentmagnet 6 angeordnet. Die Mittelachsen 7, 8 der Spulen 4, 5 verlaufen senkrecht zum die Auflagefläche des Gerätes bildenden Boden 9 des Gehäuses 1. Die Längsachsen 110, 11 der Kondensatoren 2, 3 kreuzen die Mittelachsen 7, 8 der Spulen 4, 5 rechtwinklig und verlaufen somit parallel zum Boden 9 des Gehäuses.

Um das Gerät in Betrieb zu setzen, bzw. ausser Betrieb nehmen zu können, ist jedem Schwingkreis ein Schalter 12 zugeordnet. Es kann hier ein mehrpoliger Schalter vorhanden sein, so dass mittels Betätigung, z. B. eines Druckknopfes beide Schwingkreise geschlossen, bzw.

unterbrochen werden können.

Das Gerät nach der Fig. 3 unterscheidet sich von der in den Fig. 1 und 2 gezeigten Ausführung, indem eine dritte Spule 13 vorhanden ist. Diese dritte Spule 13 ist mit dem ersten Kondensator 2 verbunden, ist also parallel zur ersten Spule 4 geschaltet. Die Achse der dritten Spule 13 fällt mit der Achse der ersten Spule 4 zusammen.

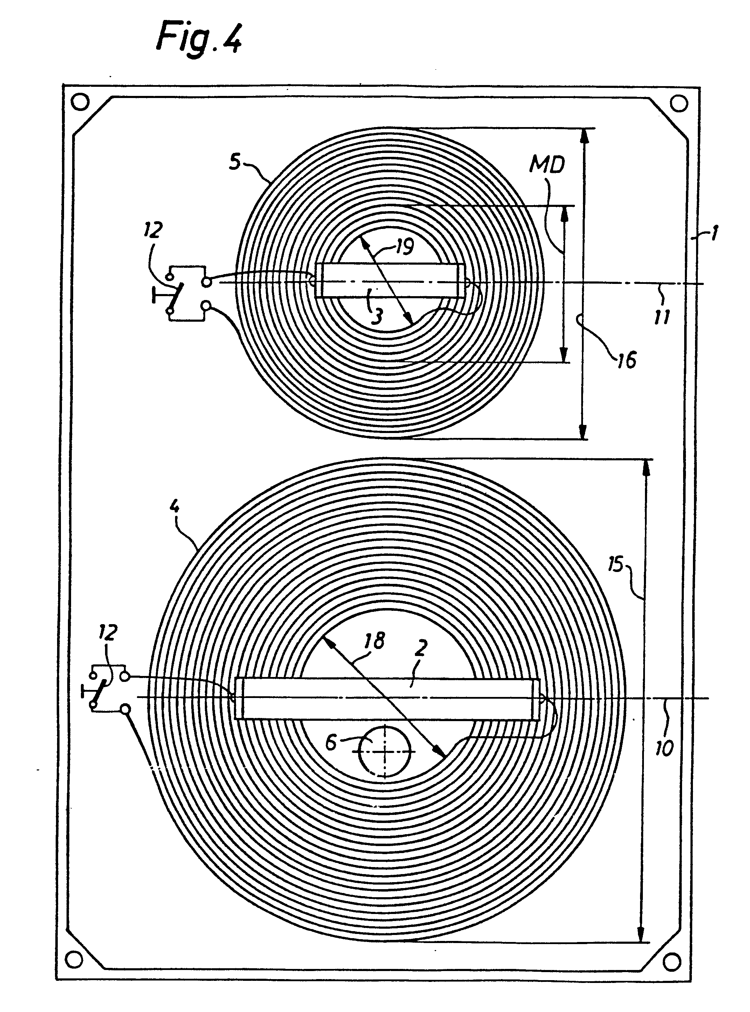

Die Spulen 2, 3 bzw. 13 der in den Figuren 1-3 gezeichneten Ausführungen sind durch gewundene Leiter gebildet. Ein solcher Leiter kann ein isolierter Draht, z. B. eine Kupferlitze oder ein lackierter Kupferdraht sein. Die Spulen können auch zum Teil durch eine gedruckte Schaltung gebildet sein, wobei eine Ausführung in der Fig. 4 gezeichnet ist. Es können alle Spulen 2, 3 und auch 13, falls eine Ausführung mit drei Spulen vorhanden ist, oder auch nur eine Spule als ebene gedruckte Schaltung gebildet sein.

In der Ausführung, die in der Fig. 4 gezeichnet ist, sind die erste Spule 4 und die zweite Spule 5 als gedruckte Schaltung gebildet. Wieder ist in der ersten Spule 4 ein Permanentmagnet angeordnet. Die Längsachsen 10, 11 der Kondensatoren 2, 3 kreuzen die Mittelachsen der Spulen 4, 5 auch hier rechtwinklig. Jedoch sind in der Ausführung nach Fig. 4 die Kondensatoren 2, 3 als auf der Spulen 4, 5 aufgelegt zu betrachten, und nicht von der Spulen 4, 5 umgeben, wie dies der Fall bei den Ausführungen gemäss der Fig. 1-3 ist.

Das Gehäuse 1 aller Ausführungen besteht aus elektrisch nicht leitendem Stoff, insbesondere aus Kunststoff, um Abschirmungen des auf das Gerät einwirkenden Erdfeldes, bzw. der Störfelder zu vermeiden. Ein Deckel 14 (siehe Fig. 3) schliesst das Gehäuse 1 ab, wobei dieser Deckel 14 zur besseren Energieaufnahme und Energieabgabe mit Löchern oder Schlitzen versehen sein kann.

Der Ausdruck "Spule" ist im Sinne einer elektrischen Induktivität zu verstehen. Die jeweiligen Kondensatoren 2, 3 bilden mit den dazugehörigen Spulen 4, 5, 13 einen praktisch dämpfungslosen elektrischen Parallelschwingkreis, der im wesentlichen dem Lenz'schen Gesetz der Physik entspricht, wonach die im Gerät ausgelöste Reaktion der sie erzeugenden Reizfeldreaktion entgegenwirkt und im Idealfall eliminiert. Die Schwingfrequenz der jeweiligen Schwingkreise liegt im allgemeinen im Bereich von 10 - 44 kHz bzw. 30 - 150 MHz. Die Grösse der Kondensatoren liegt im allgemeinen zwischen 0,1 und 2,5 μF. Die Spulen weisen jeweils mehrere Windungen auf, zumindest drei Windungen.

Die Aussendurchmesser 15, 16, 17 der Spulen 4, 5, 13 (Siehe Fig. 1 und 3) sind jeweils verschieden gross. Bei weiteren Ausführungen sind zudem die Innendurchmesser 18, 19, 20 ebenfalls jeweils verschieden gross.

Das Mass (Aussendurchmesser - Innendurchmesser = MD) des mittleren Durchmessers MD, siehe die rechts liegende Spule 5 der Fig. 1, die kleinste Spule, beträgt mindestens 5 cm.

Um zu vermeiden, dass z. B. bei Erschütterungen des Gerätes sich einzelne Bauteile im Gehäuse 1 verschieben können, ist das Gehäuse 1 vorteilhaft mit Kunstharz gefüllt. Überdies können auch das Gehäuse 1 und der Deckel 14 selbst aus Kunstharz hergestellt sein. Die Gehäusefüllung bildet eine Verstärkung der Wände des Gehäuses, wobei die einzelnen Bauteile im Kunstharz eingegossen sind.

Das Gerät ist auch dann funktionstüchtig, wenn der Kondensator nicht mehr als den mittleren Durchmesser MD der jeweiligen Spule vom Zentrum derselben entfernt ist.

Zum Einsatz des Entfeuchtungsgerätes wird zuerst bei der Einsatzstelle mittels eines elektromagnetische Wellen anzeigenden Gerätes das Störfeld geortet, bzw. seine Feldstärke ermittelt. Das Gerät wird darauf nicht unmittelbar auf die Reizstreifen eines Störfeldes oder der Stelle der höchsten Feldstärke, sondern neben einer solchen Stelle aufgestellt und durch Schliessen der Schaltkreise durch Betätigung der Schalter, bzw. des Schalters 12 in Betrieb gesetzt. Die Reichweite eines Gerätes kann bis zu mehreren Hundert Metern betragen.

Die zentrische Anordnung der Kondensatoren relativ zu den ihnen zugeordneten Spulen macht das Gerät unabhängiger von seinem Aufstellungsort und seiner Aufstellungsrichtung.

Durch die Energie der vorhandenen Störfelder werden die im Gerät angeordneten Schwingkreise erregt und erzeugen ihrerseits ein Gegenfeld. Durch die Interferenz der nun vorhandenen Felder werden diese jetzt zumindest im Bereiche der zurzeit bekannten Nachweisbarkeit so stark verringert, dass als Effekt eine messbare Verringerung der elektrokinetisch bedingten Potentialdifferenz sowie der Luftkonenkonzentration eintritt, was zur Folge hat, dass das Wasser im Mauerwerk nicht mehr aufsteigen kann und das Mauerwerk auf natürlichem Wege austrocknen kann.

**Patentansprüche**

1. Gerät zur Entfeuchtung von Mauerwerk, das Kondensatoren (2, 3) und zu Spulen (4, 5, 13) gewundene elektrische Leiter aufweist, wobei die beiden Pole eines jeweiligen Kondensators (2, 3) mit je einem Ende eines jeweiligen zur Spule (4, 5) gewundenen Leiters verbunden sind, wobei mindestens eine erste (4) und eine zweite Spule (5) und mindestens ein erster und ein zweiter Kondensator (2, 3) vorhanden sind, und der erste Kondensator (2) mit der ersten Spule (4) und der zweite Kondensator (3) mit der zweiten Spule (5) verbunden ist, die Längsachsen (10, 11) der Kondesatoren (2, 3) die jeweiligen Achsen (7, 8) der mit ihnen verbundenen Spulen (4, 5, 13) kreuzen, und die Kondensatoren (2, 3) und Spulen (4, 5) in einem Gehäuse (1) aus elektrisch nicht leitendem Stoff angeordnet sind, welches Gehäuse (1) eine Auflagefläche (9) aufweist und die Längsachse (10, 12) jedes Kondensators (2, 3) normal zur Achse (7, 8) der zugehörigen Spule (4, 5) und parallel zur Auflagefläche (9) verläuft, dadurch gekennzeichnet, dass die Achsen (7, 8) der mindestens ersten und zweiten Spule (4, 5) parallel zueinander verlaufen, wobei die Spulen (4, 5) in ihrem Aussendurchmesser (15, 16, 17) und ihrer Windungszahl verschieden sind, und dass in der Mitte mindestens einer (4) der Spulen ein Permanentmagnet angeordnet ist.

2. Gerät nach Anspruch 1, dadurch gekennzeichnet, dass die Spulen in ihrem Kerndurchmesser verschieden sind.

3. Gerät nach Anspruch 1, dadurch gekennzeichnet, dass jede Spule mindestens drei Windungen aufweist.

4. Gerät nach Anspruch 1, dadurch gekennzeichnet, dass die die Spulen bildenden elektrischen Leiter isoliert sind.

5. Gerät nach Anspruch 1, dadurch gekennzeichnet, dass die Spulen von Wicklungen aus isoliertem Draht gebildet sind.

8. Gerät nach Anspruch 1, dadurch gekennzeichnet, dass mindestens eine Spule von einer gedruckten Schaltung gebildet ist.

7. Gerät nach Anspruch 8, dadurch gekennzeichnet, dass das Gehäuse mit einem die Spulen und Kondensatoren tragenden Kunstharz ausgefüllt sind.

8. Gerät nach Anspruch 1, dadurch gekennzeichnet, dass eine dritte, mit dem ersten Kondensator verbundene und parallel zur zweiten geschalteten Spule vorhanden ist, und dass die Achsen der ersten und der dritten Spule zusammenfallen.

**Claims**

1. Apparatus for the dehumidification of masonry, which includes condensers (2, 3) and electrical conduits wound to coils (4, 5, 13), in which the two poles of a respective condenser (2, 3) are connected to one respective end of one respective conduit wound to a coil (4, 5), whereby at least a first (4) and a second (5) coil and at least a first and a second condenser (2, 3) are provided and the first condenser (2) is connected to the first coil (4) and the second condenser (3) is connected to the second coil (5), the longitudinal axis (10, 11) of the condensers (2, 3) intersect the respective axis (7, 8) of the coils (4, 5) connected thereto, and the condensers (2, 3) and coils (4, 5) are located in a casing (1) of an electrically nonconductive material, which casing (1) comprises a resting surface (9) and the longitudinal axis (10, 12) of every condenser (2, 3) extends perpendicularly to the axis (7, 8) of the corresponding coil (4, 5) and parallel to the resting surface (9), characterized in that the axis (7, 8) of the at least first and second coils (4, 5) extend parallel to each other, whereby the coils (4, 5) differ regarding their outer diameters (15, 16, 17) and number of windings, and in that a permanent magnet is located at the center of at least one (4) of the coils.

2. Apparatus of claim 1, characterized in that the coils differ from each other regarding their diameter of the core.

3. Apparatus of claim 1, characterized in that every coil has at least three windings.

4. Apparatus of claim 1, characterized in that the electrical conduits forming the coils are insulated.

5. Apparatus of claim 1, characterized in that the coils are formed of windings of insulated wire.

6. Apparatus of claim 1, characterized in that at least one coil is formed by a printed circuit.

7. Apparatus of claim 6, characterized in that the casing is filled by a synthetic resin supporting the coils and the condensers.

8. Apparatus of claim 1, characterized in that a third coil is provided which is connected to the first condenser and connected in parallel to the second coil, and in that the axis of the first and the third coil coincide.

**Revendications**

1. Dispositif pour l'assèchement de la maçonnerie, qui comporte des condensateurs (2, 3) et des conducteurs électriques enroulés en bobines (4, 5, 13), les deux pôles de chaque condensateur (2, 3) étant reliés chaque fois à une extrémité d'un conducteur enroulé en bobine (4, 5), le dispositif comportat au moins une première (4) et une deuxième (5) bobine, et au moins un premier et un deuxième condensateur (2, 3), et le premier condensateur (2) étant relié à la première bobine (4) et le deuxième condensateur (3) étant relié à la deuxième bobine (5), les axes longitudinaux (10, 11) des condensateurs (2, 3) croisant chaque fois les axes (7, 8) des bobines (4, 5, 13) qui leur sont reliés, et les condensateurs (2, 3) et les bobines (4, 5) étant disposés dans un boîtier (1) en matière non conductrice de l'électricité, lequel boîtier (1) présente une surface d'assise (9) et l'axe longitudinal (10, 12) de chaque condensateur (2, 3) s'étendant normlement à l'arc (7, 8) de la bobine (4, 5) correspondante et parallèlement à la surface d'assise (9), caractérisé en ce que les axes (7, 8) d'au moins la première et deuxième bobine (4, 5) s'étendent parallèlement l'un à l'autre, les bobines (4, 5) ayant des diamètres extérieurs (15, 16, 17) différents et un nombre de tours différent, et en ce qu'au milieu d'au moins une (4) des bobines est disposé un aimant permanent.

2. Dispositif suivant la revendication 1, caractérisé en ce que les bobines ont des diamètres de noyau différents.

3. Dispositif suivant la revendication 1, caractérisé en ce que chaque bobine comprend au moins trois spires.

4. Dispositif suivant la revendication 1, caractérisé en ce que les conducteurs qui forment les bobines sont isolés.

5. Dispositif suivant la revendication 1, caractérisé en ce que les bobines sont faites de spires de fil métallique isolé.

6. Dispositif suivant la revendication 1, caractérisé en ce qu'au moins une des bobines est faite d'un circuit imprimé.

7. Dispositif suivant la revendication 6, caractérisé en ce que le boîtier est rempli d'une résine syntétique qui porte les bobines et les condensateurs.

8. Dispositif suivant la revendication 1, caractérisé en ce qu'il comprend une troisième bobine reliée au premier condensateur et parallèle à la deuxième bobine branchée, et en ce que les axes de la première et de la troisième bobine coïncident.

0 152 510

# Fig.1

1

*Fig.2*

*Fig.3*

0152510

# Fig.4